# EUROPEAN PATENT APPLICATION

(11) **EP 1 927 367 A1**
(43) Date of publication of application: **04.06.2008**
(21) Application number: 06746578.1
(22) Date of filing: 18.05.2006
(51) Int. Cl.: A61K 39/395, A61K 38/21, A61P 1/04, A61P 1/16, A61P 3/10, A61P 7/06, A61P 17/06, A61P 21/04, A61P 25/00, A61P 29/00, A61P 35/00, A61P 35/02, A61P 37/02, A61P 43/00

(54) **NOVEL PHARMACEUTICAL USING ANTI-HLA ANTIBODY**

(30) Priority: 18.05.2005 JP 2005146094
(71) Applicant: The University of Tokushima, Tokushima-shi, Tokushima 770-8501 (JP); CHUGAI SEIYAKU KABUSHIKI KAISHA, Tokyo, 115-8543 (JP)
(72) Inventor: MATSUMOTO, Toshio c/o THE UNIVERSITY OF TOKUSHIMA, Tokushima-shi, Tokushima 7708503 (JP); OZAKI, Shuji, c/o THE UNIVERSITY OF TOKUSHIMA, Tokushima-shi, Tokushima 7708503 (JP); ABE, Masahiro, c/o THE UNIVERSITY OF TOKUSHIMA, Tokushima-shi, Tokushima 7708503 (JP)
(74) Representative: Vossius & Partner
(86) International application number: PCT/JP2006/309890
(87) International publication number: WO 2006/123724

(57) **Abstract**

The present inventors tested the effects of interferons on the expression of HLA-A in cells and cell injury caused by anti-HLA class I antibodies, by using a 2D7-DB antibody as an anti-HLA class I antibody and the IM-9 cell line as the cells to be tested. As a result, the expression of HLA-A was demonstrated to be upregulated by IFNα or IFNγ. Furthermore, WST-8 assays were performed to examine the cell viability when an interferon and an anti-HLA class I antibody were used in combination. As a result, cell death activity was found to be increased by the combination of 2D7-DB and IFNα or IFNγ.

## Description

### Technical Field

The present invention relates to enhancement of anti-HLA class I antibody actions by interferons.

### Background Art

The HLA class I antigen is formed by a heterodimer of a 45-KD α chain comprising three domains (α1, α2, α3), and a 12-KD β2 microglobulin. The main role of the HLA molecule is to present CD8⁺T cells with antigenic peptides formed from about eight to ten amino acids and produced inside cells. As such, it plays a very important role in the immune response and immune tolerance induced by this peptide presentation. HLAs can be classified into class I and class II. As class I, HLA-A, B, C, and such are known to exist (hereinafter, HLA-A may be referred to as "HLA class IA").

Cell growth-suppressing and cell death-inducing effects have been observed in lymphocytes upon HLA class IA antigen ligation by antibody, suggesting that HLA molecules may also be signal transduction molecules.

More specifically, for example, there are reports showing cell growth suppression of activated lymphocytes by the B9.12.1 antibody against the α1 domain of human HLA class IA, the W6/32 antibody against the α2 domain, and the TP25.99 and A1.4 antibodies against the α3 domain (Non-patent Documents 1 and 2). Furthermore, two types of antibodies, MoAb90 and YTH862, against the human HLA class IAα1 domain have been reported to induce apoptosis in activated lymphocytes (Non-patent Documents 2, 3, and 4). Apoptosis induced by these two antibodies has been shown to be a caspase-mediated reaction (Non-patent Document 4), and therefore, HLA class IA antigens expressed in lymphocytes are also speculated to be involved in apoptosis signal transduction.

Furthermore, the 5H7 antibody against the α3 domain of human HLA class IA (Non-patent Document 5), and the RE2 antibody against the α2 domain of mouse MHC class IA (Non-patent Document 6) have also been reported to induce cell death in activated lymphocytes and the like.

The 2D7 monoclonal antibody obtained by immunizing human myeloma cells also recognizes HLA class I, and it reportedly induces strong cell death in human myeloma cell lines in a short period of time when converted into a minibody (low-molecular-weight antibody) (a diabody). The 2D7 diabody demonstrates strong cell death-inducing activity in a variety of human myeloma cell lines and activated lymphocytes, and also demonstrates a significant life-prolonging effect in multiple myeloma model mice produced by transplanting a human myeloma cell line into mice; therefore, development of this into a therapeutic agent for myeloma is being promoted (Patent Documents 1 and 2, and Non-Patent Document 7; Patent Document 2 was not yet published at the time when the basic application was filed). It is expected that further development of such treatments using cell death induction involving HLA class I will lead to development of pharmaceuticals that are highly effective against myelomas and such. However, other than the production of anti-HLA class I molecule antibodies and reduction of their molecular weight, novel pharmaceuticals as mentioned above that use cell death induction have not been reported.

Documents relating to the invention of this application are shown below.
[Patent Document 1] WO 2004/033499
[Patent Document 1] WO 2005/056603
[Non-patent Document 1] Fayen et al., Int. Immunol. 10: 1347-1358(1998)
[Non-patent Document 2] Genestier et al., Blood 90: 3629-3639 (1997)
[Non-patent Document 3] Genestier et al., Blood 90: 726-735 (1997)
[Non-patent Document 4] Genestier et al., J. Biol. Chem. 273: 5060-5066 (1998)
[Non-patent Document 5] Woodle et al., J. Immunol. 158: 2156-2164 (1997)
[Non-patent Document 6] Matsuoka et al., J. Exp. Med. 181: 2007-2015 (1995)
[Non-patent Document 7] Kimura et al., Biochem. Biophys. Res. Commun. 325: 1201-1209 (2004)

### Disclosure of the Invention

### [Problems to be Solved by the Present Invention]

The present invention was achieved in view of the above circumstances. An objective of the present invention is to produce novel pharmaceuticals that use anti-HLA class I antibodies.

### [Means for Solving the Problems]

The present inventors conducted dedicated research to solve the above-mentioned problems. It is known that cell surface expression of HLA class I is enhanced by stimulation with an interferon (hereinafter, may also be referred to as "IFN"). The present inventors performed tests on the effects of interferons on the expression of HLA-A in cells, and cell injury caused by anti-HLA class I antibodies, by using a 2D7-DB antibody (2D7-Diabody antibody; Non-Patent Document 7; hereinafter, the 2D7-Diabody antibody may also be referred to as "2D7-DB") as an anti-HLA class I antibody and targeting the IM-9 cell line. As a result, the tests demonstrated that expression of HLA-A was upregulated by IFNα or IFNγ. Furthermore, WST-8 assays were performed to examine the cell viability when an interferon and an anti-HLA class I antibody are used in combination. As a result, combination of 2D7-DB with IFNα or IFNγ was found to increase death-inducing activity. Conventionally, enhancement of action of anti-HLA class I antibodies depended on techniques for reducing molecular weight; however, these novel findings enable the enhancement of anti-HLA class I antibody actions by methods completely different from molecular weight reduction. More specifically, the present invention relates to enhancement of cell death induction by anti-HLA class I antibodies, and specifically provides the following inventions:
(1) a pharmaceutical composition comprising an interferon and an anti-HLA class I antibody as active ingredients;
(2) a pharmaceutical composition comprising an anti-HLA class I antibody as an active ingredient, wherein the composition is used in combination with an interferon;
(3) a pharmaceutical composition comprising an anti-HLA class I antibody as an active ingredient, wherein the composition is administered simultaneously with an interferon;
(4) a pharmaceutical composition comprising an anti-HLA class I antibody as an active ingredient, wherein the composition is administered after administration of an interferon;
(5) the pharmaceutical composition of any one of (1) to (4), wherein the anti-HLA class I antibody is an anti-HLA-A antibody;
(6) the pharmaceutical composition of any one of (1) to (5), wherein the anti-HLA class I antibody is a minibody;
(7) a cell death-inducing agent comprising an interferon and an anti-HLA class I antibody as active ingredients;
(8) the cell death-inducing agent of (7), wherein the anti-HLA class I antibody is an anti-HLA-A antibody;
(9) the cell death-inducing agent of (7) or (8), wherein the anti-HLA class I antibody is a minibody;
(10) a pharmaceutical composition comprising an interferon, wherein the composition is used in combination with an anti-HLA class I antibody;
(11) a pharmaceutical composition comprising an interferon, wherein the composition is administered simultaneously with an anti-HLA class I antibody;
(12) a pharmaceutical composition comprising an interferon, wherein the composition is administered before administration of an anti-HLA class I antibody;
(13) an anti-HLA class I antibody potentiator comprising an interferon as an active ingredient;
(14) a method of treating and/or preventing a tumor, which comprises the step of simultaneously administering an anti-HLA class I antibody and an interferon;
(15) a method of treating and/or preventing a tumor, which comprises the step of administering an anti-HLA class I antibody and the step of administering an interferon;
(16) a method of treating and/or preventing an autoimmune disease, which comprises the step of simultaneously administering an anti-HLA class I antibody and an interferon;
(17) a method of treating and/or preventing an autoimmune disease, which comprises the step of administering an anti-HLA class I antibody and the step of administering an interferon;
(18) the treatment and/or prevention of any one of (14) to (17) mentioned above, wherein the anti-HLA class I antibody is an anti-HLA-A antibody;
(19) the method of treatment and/or prevention of any one of (14) to (18) mentioned above, wherein the anti-HLA class I antibody is a minibody;
(20) use of an anti-HLA class I antibody and an interferon for the production of the pharmaceutical composition of (1) mentioned above or the cell death-inducing agent of (7) mentioned above;
(21) the use of an anti-HLA class I antibody and an interferon of (20) mentioned above, wherein an anti-HLA-A antibody is used as the anti-HLA class I antibody;
(22) the use of an anti-HLA class I antibody and an interferon of (20) or (21) mentioned above, wherein a minibody is used as the anti-HLA class I antibody;
(23) the use of an anti-HLA class I antibody and an interferon of any one of (20) to (22) mentioned above, wherein the pharmaceutical composition or cell death-inducing agent is for treating and/or preventing a tumor;
(24) the use of an anti-HLA class I antibody and an interferon of any one of (20) to (22) mentioned above, wherein the pharmaceutical composition or cell death-inducing agent is for treating and/or preventing an autoimmune disease;
(25) use of an anti-HLA class I antibody for the production of the pharmaceutical composition of any one of (2) to (4) mentioned above;
(26) the use of an anti-HLA class I antibody of (25) mentioned above, wherein an anti-HLA-A antibody is used as the anti-HLA class I antibody;
(27) the use of an anti-HLA class I antibody of (25) or (26) mentioned above, wherein a minibody is used as the anti-HLA class I antibody;
(28) the use of an anti-HLA class I antibody of any one of (25) to (27) mentioned above, wherein the pharmaceutical composition is for treating and/or preventing a tumor;
(29) the use of an anti-HLA class I antibody of any one of (25) to (27) mentioned above, wherein the pharmaceutical composition is for treating and/or preventing an autoimmune disease;
(30) use of an interferon for the production of a potentiator for a cell death-inducing agent, wherein the cell death-inducing agent comprises an anti-HLA class I antibody;
(31) use of an interferon for the production of a potentiator for a therapeutic and/or preventive agent for a tumor, wherein the therapeutic and/or preventive agent comprises an anti-HLA class I antibody;
(32) use of an interferon for the production of a potentiator for a therapeutic and/or preventive agent for an autoimmune disease, wherein the therapeutic and/or preventive agent comprises an anti-HLA class I antibody;
(33) the use of an interferon of any one of (30) to (32) mentioned above, wherein the anti-HLA class I antibody is an anti-HLA-A antibody;
(34) the use of an interferon of any one of (30) to (33) mentioned above, wherein the anti-HLA class I antibody is a minibody;
(35) a method for inducing cell death, wherein an anti-HLA class I antibody and an interferon are used in combination;
(36) the method for inducing cell death of (35) mentioned above, wherein the anti-HLA class I antibody is an anti-HLA-A antibody;
(37) the method for inducing cell death of (35) or (36) mentioned above, wherein the anti-HLA class I antibody is a minibody; and
(38) a method for potentiating an action of an anti-HLA class I antibody, which method comprises administering an interferon.

### Brief Description of the Drawings

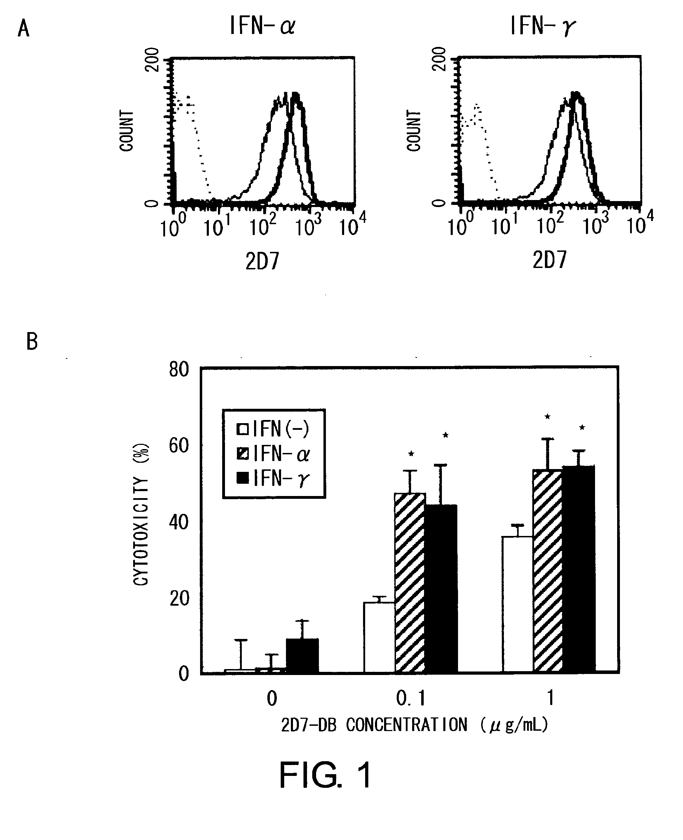

Fig. 1 shows the effects of IFNs on 2D7-DB-mediated cytotoxicity.
   (A) The effects of IFNs on HLA-A expression in myeloma cells are shown. IM-9 cells were treated with IFN-α (100 U/mL) or IFN-γ (100 U/mL) for 48 hours. Cell surface expression of HLA-A was analyzed by flow cytometry using 2D7. The staining properties of IFN-treated cells are shown by thick lines, and those of untreated cells are shown by solid lines. Negative control staining properties are shown by dotted lines. (B) A graph indicating the enhancement of 2D7-DB-mediated cytotoxicity by IFN is shown. IM-9 cells were treated with IFN-α (100 U/mL) or IFN-γ (100 U/mL) for 48 hours, and then cultured with 2D7-DB (0.1 or 1 µg/mL) for 24 hours. Cell viabilities were determined by WST-8 assays. The data show means ± SD of triplicate measurements (number of damaged cells / total number of cells) (*p < 0.05).

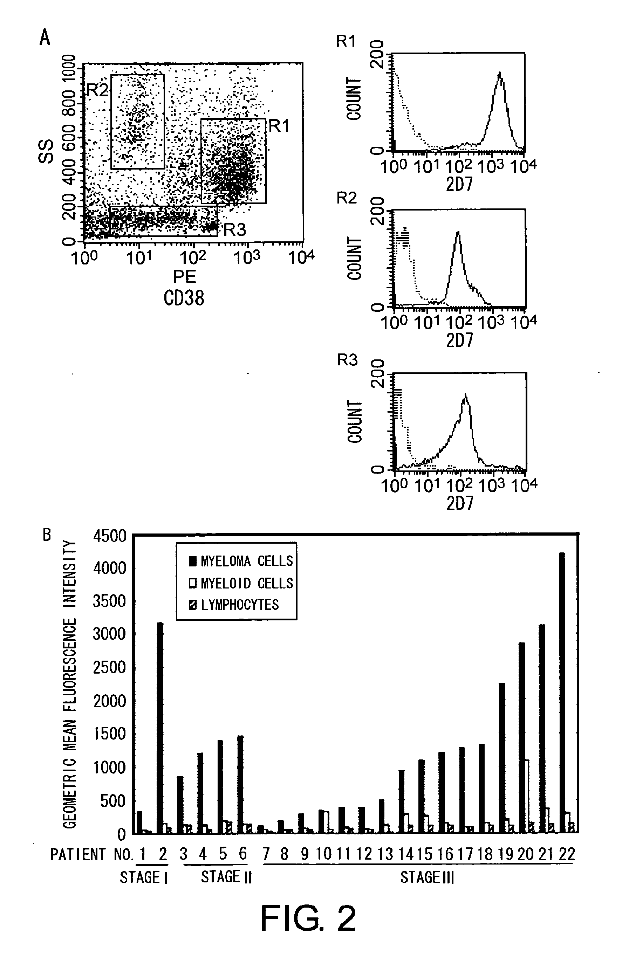

Fig. 2 shows the HLA-A expression patterns in bone marrow cells derived from multiple myeloma (MM) patients.
   (A) Bone marrow mononuclear cells (BMMCs) derived from Patient No. 17 were analyzed by flow cytometry using FITC-labeled 2D7 mAb and PE-labeled anti-CD38 mAb. After cell gating (R1: CD38-positive MM cells; R2: myeloid cells; and R3: lymphocytes), each cell population was evaluated for HLA-A expression. (B) BMMCs derived from 22 MM patients were analyzed for HLA-A expression. The data show geometric mean fluorescence intensity (GeoMFI) for each cell population.

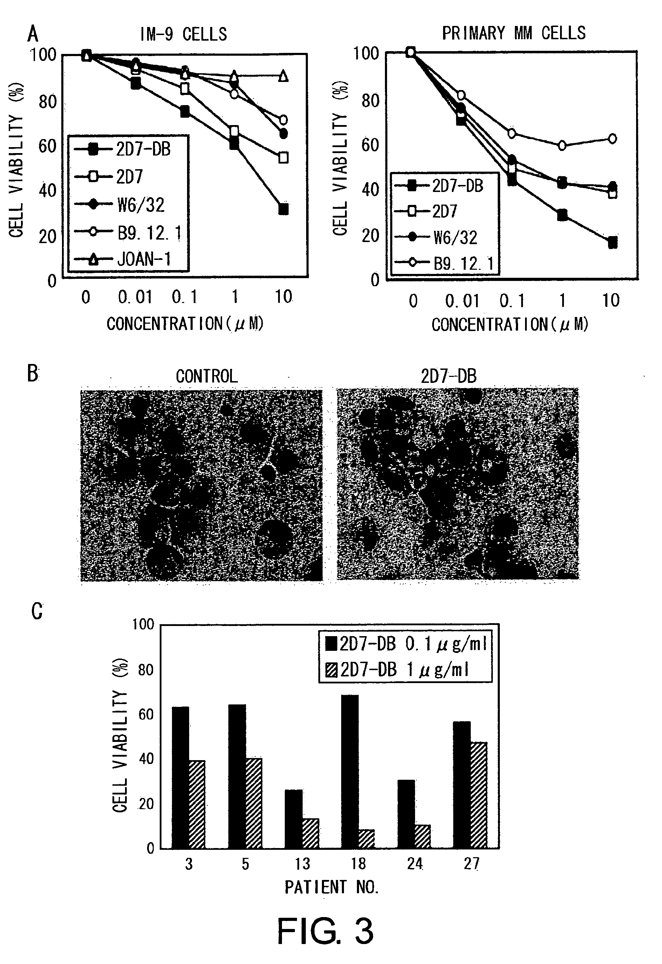

Fig. 3 shows the cytotoxic activity of anti-HLA class I antibodies on MM cells.
   (A) Graphs obtained as a result of examining the effects of various anti-HLA class I mAbs on MM cells are shown. IM-9 cells or purified MM cells derived from Patient No. 13 were cultured for 24 hours with an anti-HLA-A class I mAb, such as 2D7, W6/32, B9.12.1, and JOAN-1, and with 10 µg/mL of goat anti-mouse IgG antibody F(ab')₂. In other experiments, cells were cultured with 2D7-DB in the absence of the goat anti-mouse IgG antibody. Cell viabilities were determined by WST-8 assays or Trypan Blue exclusion assays. The data show mean values of triplicate measurements. (B) The morphological observations of myeloma cells after 2D7-DB treatment are shown. Purified MM cells derived from patient No. 18 were cultured for 24 hours in the presence (1 µg/mL) or absence of 2D7-DB. Next, cells were fixed on glass slides and stained with light Giemsa (original magnification, x 1000). (C) A graph indicating the cytotoxic activity of 2D7-DB on the patients' MM cells is shown. CD138-positive MM cells were purified from six patients, and the cells were cultured for 24 hours with 2D7-DB (0.1 or 1 µg/mL). Cell viabilities were determined by Trypan Blue exclusion assays. The data show mean values of duplicate measurements.

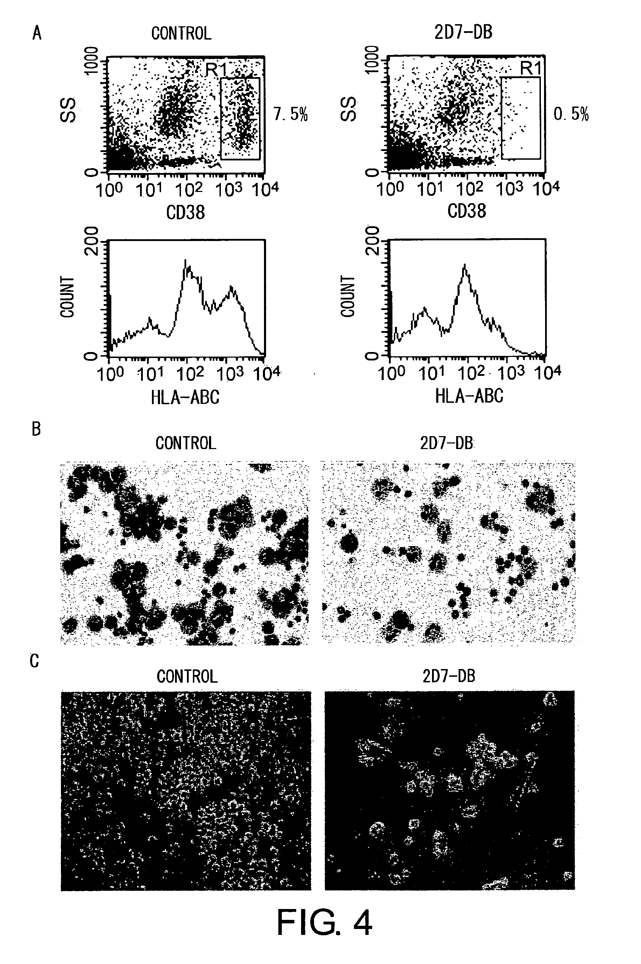

Fig. 4 shows the effects of 2D7-DB on bone marrow cells.
   (A) The eradication of a patient's MM cells by 2D7-DB is shown. Total BMMCs derived from Patient No. 12 were cultured for 48 hours in the presence (1 µg/mL) or absence of 2D7-DB. After culturing the cells, FITC-labeled anti-HLA-ABC mAb and PE-labeled anti-CD38 mAb were used to assess MM cell populations by flow cytometry. According to the side scattered light (SS) profile and CD38 expression, a gate was set for the MM cell region (R1). (B) BMMCs derived from the same patient were cultured for 48 hours in the presence (1 µg/mL) or absence of 2D7-DB. Next, cells were fixed on glass slides and stained with light Giemsa (original magnification, x 400). (C) An adherent fraction of BMMCs derived from Patient No. 12 was cultured in a 24-well plate. After stromal cells proliferated, the patient's MM cells (1 x 10⁶ cells/mL) were cultured in the same plate for 24 hours, and then subjected to 2D7-DB (1 µg/mL) treatment for 48 hours. Representative results from three independent experiments are shown.

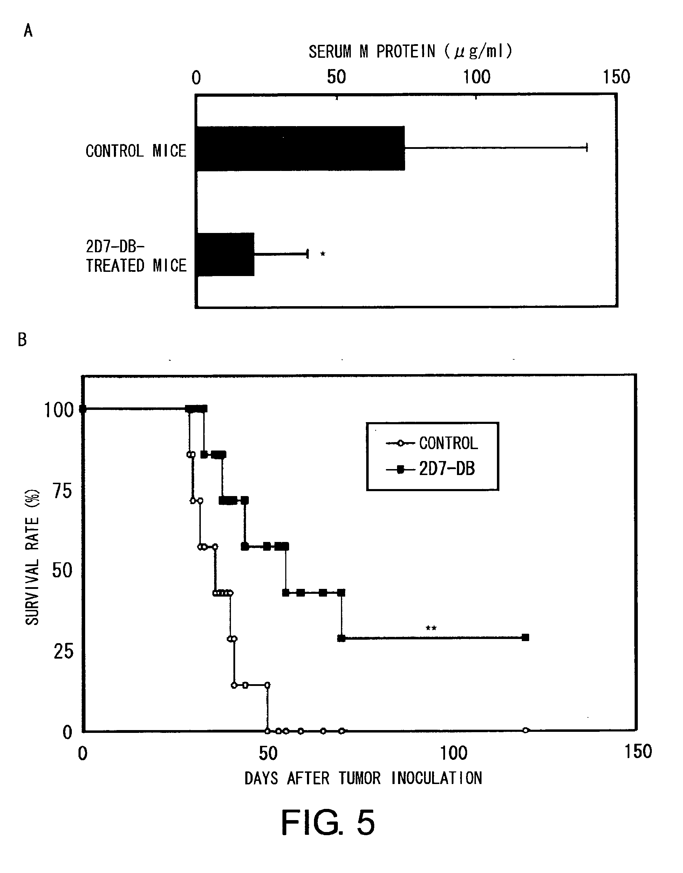

Fig. 5 shows the therapeutic efficacy of 2D7-DB in xenograft models of human myeloma. ARH-77 cells (6 x 10⁶ cells/mouse) were inoculated intravenously into SCID mice. On the first, second, and third day, a group of seven mice was treated with intravenous injection of 2D7-DB or PBS. (A) Serum concentration of human IgG was measured by ELISA on day 24. The data show means ± SD from seven mice (*p < 0.05). (B) The result of Kaplan-Meyer analysis of survival of these SCID mice is shown (**p < 0.05).

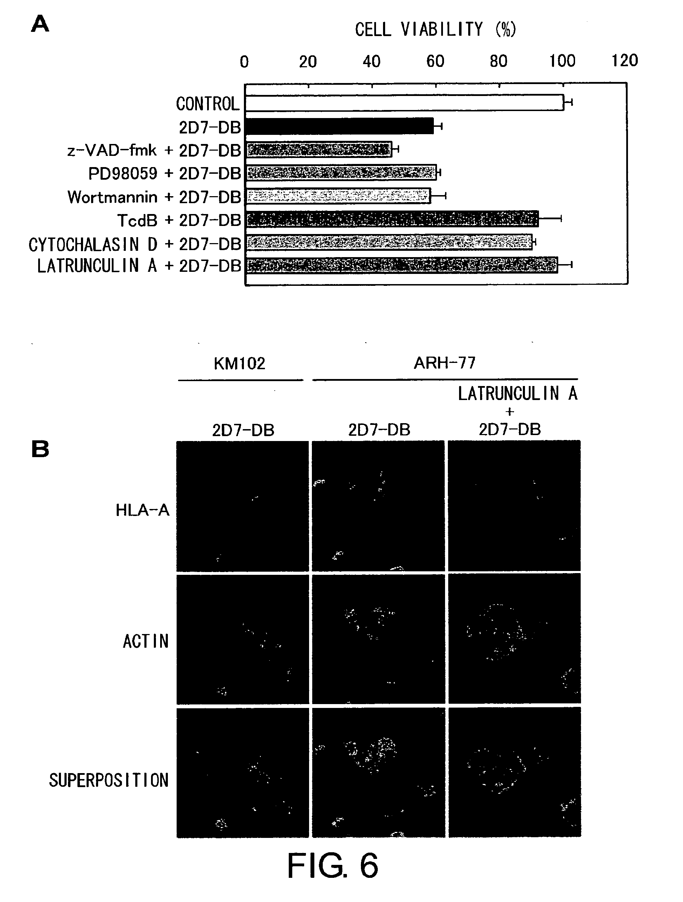

Fig. 6(A) shows the cytotoxic activity after addition of 2D7-DB to myeloma cell line RPMI 8226, which was pretreated with various signal transduction inhibitors. Treatment with a caspase inhibitor (z-VAD-fmk), MAP kinase inhibitor (PD 98059), or PI-3 kinase inhibitor (Wortmannin) did not affect the cytotoxic activity of 2D7-DB. Treatment with a Rho GTPase inhibitor (Clostridium difficile toxin B) and treatment with actin polymerization inhibitors almost completely suppressed the cytotoxic activity of 2D7-DB. Fig. 6(B) is a set of photographs showing the result of observing the localization of 2D7-DB and actin upon treatment of tumor cell lines with 2D7-DB. In the myeloma cell line ARH-77, actin aggregation took place upon 2D7 treatment, and this actin aggregation was suppressed by pretreatment with an actin polymerization inhibitor (Latrunculin A). In the bone marrow stromal cell line KM102, actin aggregation due to 2D7 treatment was not observed.

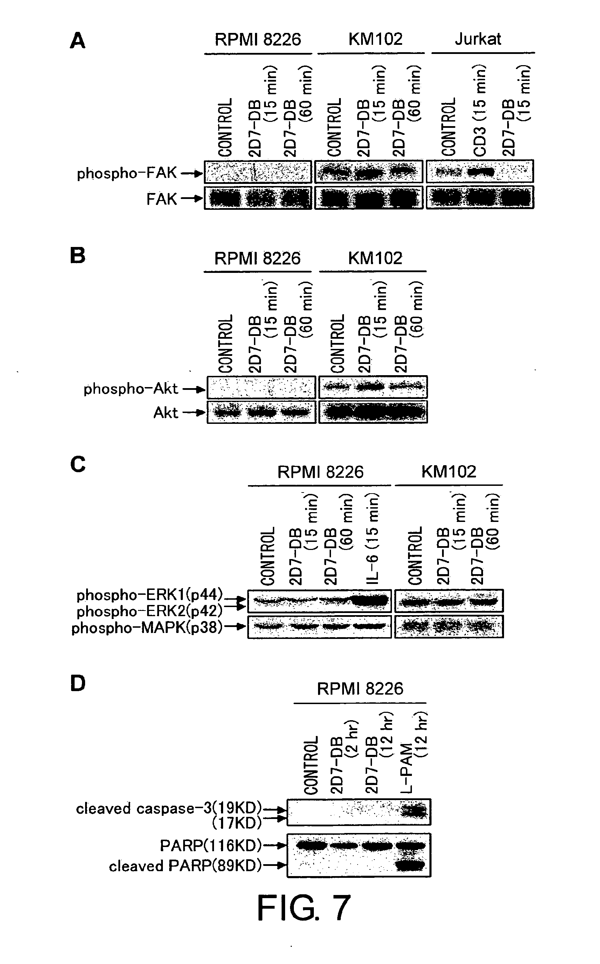

Fig. 7(A) shows the result of using Western blotting to examine signal transduction involved in cell injury by 2D7-DB. When cell lines (RPMI8226, KM102, and Jurkat) were stimulated with 2D7-DB, phosphorylated FAK was detected in the KM102 cell line. Fig. 7(B) shows the result of using Western blotting to examine signal transduction involved in cell injury by 2D7-DB. When cell lines (RPMI8226 and KM102) were stimulated with 2D7-DB, phosphorylated Akt was detected in KM102. Fig. 7(C) shows the result of using Western blotting to examine signal transduction involved in cell injury by 2D7-DB. Cell lines (RPMI8226 and KM102) were stimulated with 2D7-DB to investigate whether phosphorylated ERK1, phosphorylated ERK2, and phosphorylated MAPK could be detected. Fig. 7(D) shows the result of examining the presence or absence of apoptosis induction by 2D7-DB.

### Best Mode for Carrying Out the Invention

The present invention relates to combined use of an interferon with an anti-HLA class I antibody. The present inventors demonstrated for the first time that combined use of an interferon enhances the activity of anti-HLA class I antibodies.

Generally, interferon is a generic term for proteins or glycoproteins that have an antiviral action and are induced from animal cells by viruses, double stranded RNA, lectin, and such. In addition to the antiviral action, interferons have a cell growth-suppressing action and an immunoregulatory action. They are categorized into several types according to the cells producing them, the binding ability to specific receptors, and biological and physicochemical characteristics. The major types are α, β, and γ, and other types that are known to exist are IFNω, and IFNτ. Furthermore, 20 or more subtypes of interferon α are known to exist. At present, not only naturally-derived formulations but also various genetically recombinant type formulations, such as PEG-interferons and consensus interferons have been developed and are commercially available. An example of interferon formulations includes Pegasys (Roche) which is a PEG-interferon α2a formulation.

Interferons according to the present invention may be any one of the above-mentioned types, but are preferably α or γ. Furthermore, the interferons according to the present invention may be any one of naturally occurring forms, artificially mutated recombinant forms, naturally existing mutants, fusion proteins, or fragments thereof, so long as they can enhance cell death induction by anti-HLA class I antibodies. The interferons according to the present invention is not particularly limited in terms of their origin; the interferons can be derived from, for example, humans, chimpanzees, orangutans, dogs, horses, sheep, goats, donkeys, pigs, cats, mice, guinea pigs, rats, rabbits, or such. Without further limitation, the interferons can be derived from other mammals. Preferably, the interferons are derived from humans.

The amino acid sequence is known for human interferon α and γ, and for example, the amino acid sequence of GenBank: NM_0240013 can be used for interferon α, and the amino acid sequence of GenBank: NM_000619 can be used for interferon γ. The amino acid sequence and nucleotide sequence of interferon α are shown in SEQ ID NOs: 1 and 2 respectively, and the amino acid sequence and nucleotide sequence of interferon γ are shown in SEQ ID NOs: 3 and 4 respectively. The above-mentioned interferons can be prepared by methods well known to those skilled in the art. For example, they can be prepared by preparing mRNAs from interferon-producing cells derived from humans by a generally known technique to prepare a cDNA library; selecting from the cDNA library, cDNAs that hybridize under stringent conditions with a probe comprising all or a part of the nucleotide sequence of SEQ ID NO: 2 or 4; expressing the cDNAs using a suitable host-vector system; and purifying the obtained proteins. A host-vector system selected from the later-described examples of host-vector systems that are applicable to antibody production may be used. Alternatively, the interferons can be prepared by designing primers based on the nucleotide sequences of SEQ ID NO: 2 or 4, performing RT-PCR using mRNAs prepared from human-derived interferon producing cells as templates and using the above-mentioned primers, and then expressing the obtained cDNAs.

Those skilled in the art can appropriately select the above stringent hybridization conditions. For example, pre-hybridization is carried out in a hybridization solution containing 25% formamide, or 50% formamide under more stringent conditions, and 4x SSC, 50 mM Hepes (pH7.0), 10x Denhardt's solution, and 20 µg/ml denatured salmon sperm DNA at 42°C overnight. A labeled probe is then added to the solution and hybridization is carried out by incubation at 42°C overnight. Post-hybridization washes can be carried out with conditions for washing solution and temperature of about "1x SSC, 0.1% SDS, 37°C", or more stringent conditions of about "0.5x SSC, 0.1% SDS, 42°C", or more highly stringent conditions of about "0.2x SSC, 0.1% SDS, 65°C". As the stringency of the post-hybridization washes increases, polynucleotides with greater homology to the probe sequence are expected to be isolated. The above-described combinations of SSC, SDS, and temperature are merely examples of washing conditions. Those skilled in the art can achieve the same stringencies as those described above by appropriately combining the above factors or others (such as probe concentration, probe length, or hybridization period) that affect hybridization stringency.

Polypeptides encoded by polynucleotides isolated using such hybridization techniques will usually comprise amino acid sequences highly homologous to the polypeptides identified by the present inventors. "High homology" refers to sequence homology of at least 40% or more, preferably 60% or more, further preferably 80% or more, further preferably 90% or more, further preferably at least 95% or more, and further preferably at least 97% or more (for example, 98% to 99%). Amino acid sequence identity can be determined, for example, using the BLAST algorithm of Karlin and Altschul (Proc. Natl. Acad. Sci. USA. 87:2264-2268, 1990; Proc. Natl. Acad. Sci. USA 90: 5873-5877, 1993). A program called BLASTX has been developed based on this algorithm (Altschul et al., J. Mol. Biol. 215: 403-410, 1990). When using BLASTX to analyze amino acid sequence identity, the parameters are, for example, a score of 50 and a word length of 3. When using the BLAST and Gapped BLAST programs, the default parameters for each program are used. Specific methodology for these analysis methods is well known (http://www.ncbi.nlm.nih.gov).

Furthermore, without limitation to the interferons of the above-mentioned sequences, polypeptides similar to the interferons of the above-mentioned sequences can also be suitably used in the present invention so long as they enhance cell death induction by anti-HLA class I antibodies. Examples of such polypeptides include a polypeptide that has an effect to enhance cell death induction by an anti-HLA class I antibody and comprises an amino acid sequence with one or more amino acid deletions, substitutions, additions, and/or insertions in the amino acid sequence of SEQ ID NO: 1; a polypeptide that has an effect to enhance cell death induction by anti-HLA class I antibody and comprises an amino acid sequence with one or more amino acid deletions, substitutions, additions, and/or insertions in the amino acid sequence of SEQ ID NO: 3; a polypeptide that has an effect to enhance cell death induction by anti-HLA class I antibody and comprises an amino acid sequence encoded by a nucleotide sequence that hybridizes under stringent conditions with the nucleotide sequence of SEQ ID NO: 2; and a polypeptide that has an effect to enhance cell death induction by anti-HLA class I antibody and comprises an amino acid sequence encoded by a nucleotide sequence that hybridizes under stringent conditions with the nucleotide sequence of SEQ ID NO: 4.

Such polypeptides can be prepared by methods well known to those skilled in the art. For example, all or a portion of the nucleotide sequence of SEQ ID NO: 2 or 4 can be used as a probe to select hybridizing clones from a cDNA library prepared from interferon-producing cells, and the clones can be expressed to prepare the polypeptides. Alternatively, the polypeptides can be prepared by performing gene modification methods well known to those skilled in the art, such as PCR mutagenesis or cassette mutagenesis, on the nucleotide sequence of SEQ ID NO: 2 or 4 to site-specifically or randomly introduce mutations. It is also possible to synthesize sequences with mutations which have been introduced into the nucleotide sequence of SEQ ID NO: 2 or 4, by using a commercially available nucleic acid synthesizer.

Whether or not polypeptides prepared in this manner will enhance cell death induction by anti-HLA class I antibodies can be assessed by publicly known methods. For example, after contacting a test polypeptide with HLA class I-expressing cells, cell viability of these cells can be assessed by WST-8 assays, Trypan blue exclusion assays, or such. More specifically, the assessment can be performed by the method described in Reference Example 2 of the present description.

In the present invention, anti-HLA class I antibodies are antibodies that recognize molecules categorized into HLA class I. In the present invention, HLA refers to human leukocyte antigen. HLA molecules are categorized into class I and class II. Known examples of class I are HLA-A, B, C, E, F, G, H, J, and such; and known examples of class II are HLA-DR, DQ, DP, and such. Antigens recognized by anti-HLA class I antibodies in the present invention are not particularly limited so long as they are molecules classified as HLA class I; however, the antigens are preferably HLA-A. In the present invention, leukocytes are blood cells involved in biological defense. Generally, leukocytes include lymphocytes (B-cells, helper T-cells, suppressor T-cells, killer T-cells, and NK cells), monocytes (macrophages), granulocytes (neutrophils, eosinophils, and basophils), and such. In the context of the present invention, leukocytes may be any of the leukocytes including lymphocytes (B-cells, helper T-cells, suppressor T-cells, killer T-cells, and NK cells), monocytes (macrophages), granulocytes (neutrophils, eosinophils, and basophils), and such.

In the present invention, HLA class I-recognizing antibodies are not particularly limited so long as they recognize HLA class I; however, antibodies that specifically recognize HLA class I are preferred.

The HLA class I-recognizing antibodies may be well known antibodies, or may be anti-HLA class I antibodies prepared by a method well known to those skilled in the art using HLA class I as antigens. More specifically, the antibodies can be prepared as described below.

The HLA class I protein or a fragment thereof is used as a sensitizing antigen to perform immunization according to conventional immunization methods, the obtained immunocytes are fused with known parent cells according to conventional cell fusion methods, and monoclonal antibody-producing cells (hybridomas) are then screened by general screening methods. Antigens can be prepared by known methods, such as methods using baculoviruses (WO98/46777 and such). Hybridomas can be prepared according to the method of Milstein *et al.* (Kohler, G. and Milstein, C., Methods Enzymol. (1981) 73:3-46), for example. When an antigen has low immunogenicity, immunization can be performed by binding the antigen to an immunogenic macromolecule such as albumin. Then, cDNAs of the antibody variable region (V region) are synthesized from the mRNAs of the hybridomas using reverse transcriptase, and the sequences of the obtained cDNAs can be determined by known methods.

Anti-HLA class I antibodies are not particularly limited in terms of their origin, so long as they bind to HLA. Mouse antibodies, rat antibodies, rabbit antibodies, sheep antibodies, human antibodies, and such may be used as necessary. Alternatively, artificially modified genetically recombinant antibodies, such as chimeric and humanized antibodies, may be used to reduce heterologous antigenicity against humans. These modified antibodies can be produced using known methods. A chimeric antibody is an antibody comprising the heavy and light chain variable regions of an antibody from a non-human mammal such as mouse, and the heavy and light chain constant regions of a human antibody. The chimeric antibody can be produced by linking a DNA encoding mouse antibody variable regions with a DNA encoding human antibody constant regions, incorporating this into an expression vector, and then introducing the vector into a host.

Humanized antibodies are also referred to as "reshaped human antibodies". Such humanized antibodies are obtained by grafting the complementarity determining region (CDR) of an antibody derived from a non-human mammal, for example, a mouse, to the CDR of a human antibody. General gene recombination procedures for humanized antibody production are also known. Specifically, a DNA sequence designed to link a murine antibody CDR to the framework region (FR) of a human antibody is synthesized by PCR, using several oligonucleotides produced to contain overlapping portions in the terminal regions. The obtained DNA is linked to a DNA encoding a human antibody constant region, and this is then integrated into an expression vector, and the antibody is produced by introducing this vector into a host (see European Patent Application Publication No. EP 239400, and International Patent Application Publication No. WO 96/02576). The human antibody FR to be linked via CDR is selected so that the CDR forms a favorable antigen-binding site. In order for the CDR of the reshaped human antibody to form a suitable antigen-binding site, the amino acids in the framework region of the antibody variable region may be substituted as necessary (Sato, K. et al., 1993, Cancer Res. 53, 851-856).

Methods for obtaining human antibodies are also known. For example, human lymphocytes can be sensitized *in vitro* with a desired antigen, or with cells expressing a desired antigen, and the sensitized lymphocytes can be fused with human myeloma cells such as U266, to obtain the desired human antibody with antigen-binding activity (see Japanese Patent Application Kokoku Publication No. (JP-B) Hei 1-59878 (examined, approved Japanese patent application published for opposition)). Further, a desired human antibody can be obtained by using a desired antigen to immunize transgenic animals that have a full repertoire of human antibody genes (see International Patent Application Publication Nos. WO 93/12227, WO 92/03918, WO 94/02602, WO 94/25585, WO 96/34096, and WO 96/33735). Furthermore, techniques for obtaining human antibodies by panning using a human antibody library are also known. For example, variable regions of human antibodies can be expressed as single-chain antibodies (scFvs) on the surface of phages using phage display methods, and phages that bind to antigens can be selected. DNA sequences encoding the variable regions of human antibodies that bind to the antigens can be determined by analyzing the genes of the selected phages. By revealing the DNA sequences of the scFvs that bind to the antigens, appropriate expression vectors comprising the sequences can be produced to yield human antibodies. These methods are already known, and the following publications can be referred to: WO 92/01047, WO 92/20791, WO 93/06213, WO 93/11236, WO 93/19172, WO 95/01438, and WO 95/15388.

As anti-HLA class I antibodies preferred in the present invention, anti-HLA class I antibodies that have been converted into minibodies can be raised. Minibodies include antibody fragments in which part of a whole antibody (for example, whole IgG) is missing, and are not particularly limited so long as they have antigen-binding ability. Antibody fragments of the present invention are not particularly limited so long as they are part of a whole antibody. However, fragments comprising heavy chain variable regions (VH) or light chain variable regions (VL) are preferred, and fragments comprising both VH and VL are particularly preferred. Specific examples of antibody fragments include Fab, Fab', F(ab')2, Fv, scFv (single chain Fv), and such, but are preferably scFvs (Huston, J. S. et al., Proc. Natl. Acad. Sci. U.S.A. (1988) 85, 5879-5883; Plickthun "The Pharmacology of Monoclonal Antibodies" Vol.113, Resenburg and Moore ed., Springer Verlag, New York, pp.269-315, (1994)). Such antibody fragments can be obtained by treating an antibody with enzymes such as papain or pepsin to produce antibody fragments, or by constructing genes encoding such antibody fragments, introducing them into an expression vector, and then expressing this in an appropriate host cell (see for example, Co, M. S. et al., J. Immunol. (1994) 152, 2968-2976; Better, M. and Horwitz, A. H., Methods Enzymol. (1989) 178, 476-496; Pluckthun, A. and Skerra, A., Methods Enzymol. (1989) 178, 497-515; Lamoyi, E., Methods Enzymol. (1986) 121, 652-663; Rousseaux, J. et al., Methods Enzymol. (1986) 121, 663-669; Bird, R. E. and Walker, B. W., Trends Biotechnol. (1991) 9, 132-137).

The molecular weight of a minibody of the present invention is preferably smaller than that of the whole antibody, but multimers such as dimers, trimers, and tetramers may be formed, in which case the molecular weight can be larger than the molecular weight of the whole antibody.

Minibodies preferred in the present invention are those antibodies that comprise two or more antibody VHs and two or more antibody VLs, in a form that each of these variable regions is linked directly or indirectly via linkers or such. The linkages may be covalent bonds or non-covalent bonds, or may be the both. As even more preferable minibodies, antibodies comprising two or more VH-VL pairs that are formed by non-covalent bonds between VH and VL are raised. In this case, the distance between one VH-VL pair and another VH-VL pair is preferably shorter in a minibody than in a whole antibody.

In the present invention, particularly preferable minibodies are diabodies or sc(Fv)2. A diabody is a dimer formed by linking two fragments (for example, scFv) (hereinafter referred to as diabody-constituting fragments), in which a variable region is linked to another variable region via a linker or such, and usually comprises two VLs and two VHs. The bonds between the diabody-constituting fragments may be non-covalent or covalent bonds, but are preferably non-covalent bonds.

Diabody-constituting fragments include those with linked VL-VH, VL-VL, VH-VH, and such, and are preferably those with linked VH-VL. In diabody-constituting fragments, the linker used to link a variable region to a variable region is not particularly limited, but is preferably a linker short enough to prevent non-covalent bonding between variable regions in the same fragment. The length of such a linker can be suitably determined by those skilled in the art, and is ordinarily 2 to 14 amino acids, preferably 3 to 9 amino acids, and most preferably 4 to 6 amino acids. In this case, linkers between VL and VH encoded on a same fragment are short, and thus VL and VH on a same strand do not form a non-covalent bond and therefore a single-chain V region fragment will not be formed. Rather, a fragment forms a dimer with another fragment via non-covalent bonding. Furthermore, according to the same principle in diabody construction, three or more diabody-constituting fragments may be linked to form multimeric antibodies such as trimers and tetramers.

Without limitation, examples of the diabodies in the present invention include a diabody comprising the amino acid sequence of SEQ ID NO: 5; a diabody that comprises an amino acid sequence with one or more amino acid sequence mutations (substitutions, deletions, insertions, and/or additions) in the amino acid sequence of SEQ ID NO: 5 and that is functionally equivalent to the diabody comprising the sequence of SEQ ID NO: 5; a diabody comprising the amino acid sequences of the CDR (or the variable region) of SEQ ID NO: 6 and the CDR (or the variable region) of SEQ ID NO: 7; and a diabody that comprises an amino acid sequence with one or more amino acid sequence mutations (substitutions, deletions, insertions, and/or additions) in the amino acid sequences of the CDR (or the variable region) of SEQ ID NO: 6 and the CDR (or the variable region) of SEQ ID NO: 7 and is functionally equivalent to the diabody comprising the sequences of the CDR (or the variable region) of SEQ ID NO: 6 and the CDR (or the variable region) of SEQ ID NO: 7.

Herein, being "functionally equivalent" means that the diabody of interest has an activity equivalent to that of a diabody comprising the sequence of SEQ ID NO: 5, or a diabody comprising the sequences of the CDR (or the variable region) of SEQ ID NO: 6 and the CDR (or the variable region) of SEQ ID NO: 7 (examples of the activity include HLA-A binding activity, cell death-inducing activity, and such).

The number of mutated amino acids is not particularly limited, but may usually be 30 amino acids or less, preferably 15 amino acids or less, and more preferably five amino acids or less (for example, three amino acids or less).

Furthermore, a diabody comprising the amino acid sequence of SEQ ID NO: 5, or a diabody comprising the sequences of the CDR (or the variable region) of SEQ ID NO: 6 and the CDR (or the variable region) of SEQ ID NO: 7 may be humanized or chimerized to reduce heterologous antigenicity against humans.

In the amino acid sequence of SEQ ID NO: 6, amino acids 1 to 134 correspond to the variable region, amino acids 50 to 54 correspond to CDR1, amino acids 69 to 85 correspond to CDR2, and amino acids 118 to 134 correspond to CDR3. In the amino acid sequence of SEQ ID NO: 7, amino acids 1 to 128 correspond to the variable region, amino acids 46 to 55 correspond to CDR1, amino acids 71 to 77 correspond to CDR2, and amino acids 110 to 128 correspond to CDR3.

sc(Fv)2 is a single-chain polypeptide antibody, prepared by linking two sets of heavy chain variable regions ([VH]) and two sets of light chain variable regions ([VL]) with linkers and such (Hudson et al., J. Immunol. Methods 1999; 231: 177-189). sc(Fv)2 can be prepared, for example, by linking two scFv (single chain Fv) molecules (Huston, J. S. et al., Proc. Natl. Acad. Sci. U.S.A. (1988) 85, 5879-5883; Plickthun "The Pharmacology of Monoclonal Antibodies" Vol.113, Resenburg and Moore ed., Springer Verlag, New York, pp.269-315, (1994)) with a linker and such. As linkers, arbitrary peptide linkers that can be introduced by genetic engineering, and synthetic linkers, for example, the linkers disclosed in Protein Engineering, 9(3), 299-305, 1996 may be used, and in the present invention, peptide linkers are preferred. The length of the polypeptide linkers is not particularly limited and can be suitably selected according to the purpose by those skilled in the art. Normally, the length is 1 to 100 amino acids, preferably 5 to 30 amino acids, and particularly preferably 12 to 18 amino acids (for example, 15 amino acids).

The order of the two sets of heavy chain variable region and the two sets of light chain variable region to be linked is not particularly limited and may be any order, including for example, the following arrangements.
[VH] linker [VL] linker [VH] linker [VL]
[VL] linker [VH] linker [VH] linker [VL]
[VH] linker [VL] linker [VL] linker [VH]
[VH] linker [VH] linker [VL] linker [VL]
[VL] linker [VL] linker [VH] linker [VH]
[VL] linker [VH] linker [VL] linker [VH]

In the context of the present invention, a preferred sc(Fv)2 arrangement is [VH] linker [VL] linker [VH] linker [VL].

The amino acid sequence of the heavy chain variable region or the light chain variable region may contain substitutions, deletions, additions, and/or insertions. Furthermore, it may also lack portions of heavy chain variable region and/or light chain variable region, or other polypeptides may be added, as long as the binding complex of heavy chain variable regions and light chain variable regions retains its antigen binding activity. Additionally, the variable region may be chimerized or humanized.

In the present invention, the linkers to be used for linking the variable regions of an antibody include arbitrary peptide linkers that can be introduced by genetic engineering, synthetic linker compounds, for example, those disclosed in Protein Engineering, 9(3), 299-305, 1996.

In the present invention, preferred linkers are peptide linkers. The length of the polypeptide linkers is not particularly limited and can be suitably selected according to the purpose by those skilled in the art. Normally, the length is 1 to 100 amino acids, preferably 3 to 50 amino acids, more preferably 5 to 30 amino acids, and even more preferably 12 to 18 amino acids (for example, 15 amino acids).

For example, amino acid sequences for such peptide linkers include:
Ser
Gly·Ser
Gly·Gly·Ser
Ser·Gly·Gly
Gly·Gly·Gly·Ser (SEQ ID NO: 22)
Ser·Gly·Gly·Gly (SEQ ID NO: 23)
Gly·Gly·Gly·Gly·Ser (SEQ ID NO: 24)
Ser·Gly·Gly·Gly·Gly (SEQ ID NO: 25)
Gly·Gly·Gly·Gly·Gly·Ser (SEQ ID NO: 26)
Ser·Gly·Gly·Gly·Gly·Gly (SEQ ID NO: 27)
Gly·Gly·Gly·Gly·Gly·Gly·Ser (SEQ ID NO: 28)
Ser·Gly·Gly·Gly·Gly·Gly·Gly (SEQ ID NO: 29)
(Gly·Gly·Gly·Gly·Ser [SEQ ID NO: 24])n
(Ser·Gly·Gly·Gly·Gly [SEQ ID NO: 25])n
where n is an integer of 1 or more.

Synthetic linker compounds (chemical crosslinking agents) include, crosslinking agents routinely used to crosslink peptides, for example, *N*-hydroxysuccinimide (NHS), disuccinimidyl suberate (DSS), bis(sulfosuccinimidyl)suberate (BS3), dithiobis(succinimidyl propionate) (DSP), dithiobis(sulfosuccinimidyl propionate) (DTSSP), ethylene glycol bis(succinimidyl succinate) (EGS), ethylene glycol bis(sulfosuccinimidyl succinate) (sulfo-EGS), disuccinimidyl tartarate (DST), disulfosuccinimidyl tartarate (sulfo-DST), bis[2-(succinimidooxycarbonyloxy)ethyl]sulfone (BSOCOES), and bis[2-(sulfosuccinimidooxycarbonyloxy)ethyl]sulfone (sulfo-BSOCOES). These crosslinking agents are commercially available.

In general, three linkers are required to link four antibody variable regions together. The linkers to be used may be of the same type or different types.

Examples of a preferred sc(Fv)2 of the present invention include, but are not limited to, any one of (a) to (i) indicated below.
(a) a sc(Fv)2 comprising heavy chain variable regions that comprise CDR1, 2, and 3 consisting of the amino acid sequences of SEQ ID NOs: 10, 11, and 12.
(b) a sc(Fv)2 comprising light chain variable regions that comprise CDR1, 2, and 3 consisting of the amino acid sequences of SEQ ID NOs: 13, 14, and 15.
(c) a sc(Fv)2 comprising heavy chain variable regions and light chain variable regions, both of which comprise CDR1, 2, and 3 consisting of the amino acid sequences of SEQ ID NOs: 10, 11, and 12, and SEQ ID NOs: 13, 14, and 15, respectively.
(d) a sc(Fv)2 comprising heavy chain variable regions comprising the amino acid sequence of SEQ ID NO: 17.
(e) a sc(Fv)2 comprising light chain variable regions comprising the amino acid sequence of SEQ ID NO: 19.
(f) a sc(Fv)2 comprising heavy chain variable regions comprising the amino acid sequence of SEQ ID NO: 17 and light chain variable regions comprising the amino acid sequence of SEQ ID NO: 19.
(g) a sc(Fv)2 comprising the amino acid sequence of SEQ ID NO: 21.
(h) a sc(Fv)2 comprising the amino acid sequence of SEQ ID NO: 9.
(i) a sc(Fv)2 comprising an amino acid sequence with one or more substitutions, deletions, additions, and/or insertions in the amino acid sequence of any one of (a) to (h), in which the sc(Fv)2 has an activity functionally equivalent to that of the antibodies of the present invention.

SEQ ID NOs: 16 and 17 correspond to the nucleotide sequence and the amino acid sequence of the 2D7 heavy chain variable region, respectively. In the amino acid sequence of SEQ ID: 17, amino acids 50 to 54 correspond to CDR1 (SEQ ID NO: 10), amino acids 69 to 85 correspond to CDR2 (SEQ ID NO: 11), and amino acids 118 to 123 correspond to CDR3 (SEQ ID NO: 12). SEQ ID NOs: 18 and 19 correspond to the nucleotide sequence and the amino acid sequence of the 2D7 light chain variable region, respectively. In the amino acid sequence of SEQ ID: 19, amino acids 46 to 55 correspond to CDR1 (SEQ ID NO: 13), amino acids 71 to 77 correspond to CDR2 (SEQ ID NO: 14), and amino acids 110 to 118 correspond to CDR3 (SEQ ID NO: 15). The nucleotide sequence of a polynucleotide encoding scFv prepared by linking the above-mentioned heavy chain variable region and light chain variable region with a linker is shown in SEQ ID NO: 20; and the amino acid sequence of this scFv is shown in SEQ ID NO: 21. The nucleotide sequence of a polynucleotide encoding the sc(Fv)2 of the present invention is shown in SEQ ID NO: 8; the amino acid sequence of this sc(Fv)2 is shown in SEQ ID NO: 9.

Furthermore, a sc(Fv)2 comprising the amino acid sequence of SEQ ID NO: 9, or a sc(Fv)2 comprising a CDR (or a variable region) in the amino acid sequence of SEQ ID NO: 9 may be humanized or chimerized to reduce heterologous antigenicity against humans. Such artificially modified antibodies can be prepared by using known methods.

Here, the term "functionally equivalent" means that the antibody of interest has an activity equivalent to the sc(Fv)2 comprising the sequence of SEQ ID NO: 9, or the sc(Fv)2 comprising a CDR (or a variable region) in the amino acid sequence of SEQ ID NO: 9 (for example, HLA-A binding activity and cell death-inducing activity).

Methods for preparing polypeptides functionally equivalent to a certain polypeptide are well known to those skilled in the art, and include methods of introducing mutations into polypeptides. For example, one skilled in the art can prepare an antibody functionally equivalent to an antibody of the present invention by introducing appropriate mutations into the antibody using site-directed mutagenesis (Hashimoto-Gotoh, T. et al. (1995) Gene 152, 271-275; Zoller, MJ, and Smith, M.(1983) Methods Enzymol. 100, 468-500; Kramer, W. et al. (1984) Nucleic Acids Res. 12, 9441-9456; Kramer W, and Fritz HJ(1987) Methods. Enzymol. 154, 350-367; Kunkel, TA (1985) Proc Natl. Acad. Sci. USA. 82, 488-492; Kunkel (1988) Methods Enzymol. 85, 2763-2766). Amino acid mutations may also occur naturally. Therefore, the antibodies of the present invention also include antibodies functionally equivalent to the antibodies of the present invention, wherein the antibodies comprises amino acid sequences with one or more amino acid mutations to the amino acid sequences of the present invention's antibodies.

The number of amino acids that are mutated is not particularly limited, but is generally 30 amino acids or less, preferably 15 amino acids or less, and more preferably 5 amino acids or less (for example, 3 amino acids or less). Preferably, the mutated amino acids conserve the properties of the amino acid side chain from the amino acids that were mutated. Examples of amino acid side chain properties include: hydrophobic amino acids (A, I, L, M, F, P, W, Y, and V), hydrophilic amino acids (R, D, N, C, E, Q, G, H, K, S, and T), amino acids comprising the following side chains: aliphatic side chains (G, A, V, L, I, and P); hydroxyl-containing side chains (S, T, and Y); sulfur-containing side chains (C and M); carboxylic acid- and amide-containing side chains (D, N, E, and Q); basic side chains (R, K, and H); and aromatic ring-containing side chains (H, F, Y, and W) (amino acids are represented by one-letter codes in parentheses). Polypeptides comprising a modified amino acid sequence, in which one or more amino acid residues is deleted, added, and/or substituted with other amino acids, are known to retain their original biological activities (Mark, D. F. et al., Proc. Natl. Acad. Sci. USA (1984) 81, 5662-5666; Zoller, M. J. & Smith, M. Nucleic Acids Research (1982) 10, 6487-6500; Wang, A. et al., Science 224, 1431-1433; Dalbadie-McFarland, G. et al., Proc. Natl. Acad. Sci. (1982) USA 79, 6409-6413). Amino acid sequences of antibody constant regions and such are known to those skilled in the art.

The antibodies to be used in the present invention may be conjugated antibodies that are bound to various molecules, including, for example, polyethylene glycol (PEG), radioactive substances, and toxins. Such conjugate antibodies can be obtained by chemically modifying the obtained antibodies. Methods for antibody modification are already established in this field. Accordingly, the term "antibody" as used herein includes such conjugate antibodies.

The above-mentioned antibodies can be produced by methods well known to those skilled in the art. More specifically, a DNA of an antibody of interest is incorporated into an expression vector. In so doing, the DNA is incorporated into the expression vector and expressed under the control of an expression regulatory region such as an enhancer or promoter. Next, antibodies can be expressed by transforming host cells with this expression vector. In this regard, appropriate combinations of hosts and expression vectors can be used.

The vectors include, for example, M13 vectors, pUC vectors, pBR322, pBluescript, and pCR-Script. In addition to the above vectors, for example, pGEM-T, pDIRECT, and pT7 can also be used for the subcloning and excision of cDNAs.

When using vectors to produce the antibodies, expression vectors are particularly useful. When an expression vector is expressed in *E. coli,* for example, it should have the above characteristics in order to be amplified in *E. coli*. Additionally, when *E. coli* such as JM109, DH5α, HB101, or XL1-Blue are used as the host cell, the vector preferably has a promoter, for example, a lacZ promoter (Ward et al. (1989) Nature 341:544-546; (1992) FASEB J. 6:2422-2427), araB promoter (Better et al. (1988) Science 240:1041-1043), or T7 promoter, to allow efficient expression of the desired gene in *E. coli.* Other examples of the vectors include pGEX-5X-1 (Pharmacia), "QIAexpress system" (QIAGEN), pEGFP, and pET (where BL21, a strain expressing T7 RNA polymerase, is preferably used as the host).

Furthermore, the vector may comprise a signal sequence for polypeptide secretion. When producing proteins into the periplasm of *E. coli,* the pelB signal sequence (Lei, S. P. et al. J. Bacteriol. 169:4379 (1987)) may be used as a signal sequence for protein secretion. For example, calcium chloride methods or electroporation methods may be used to introduce the vector into a host cell.

In addition to *E. coli,* expression vectors derived from mammals (e.g., pCDNA3 (Invitrogen), pEGF-BOS (Nucleic Acids Res. (1990) 18(17):5322), pEF, pCDM8), insect cells (e.g., "Bac-to-BAC baculovirus expression system" (GIBCO-BRL), pBacPAK8), plants (e.g., pMH1, pMH2), animal viruses (e.g., pHSV, pMV, pAdexLcw), retroviruses (e.g., pZIPneo), yeasts (e.g., "Pichia Expression Kit" (Invitrogen), pNV11, SP-Q01), and *Bacillus subtilis* (e.g., pPL608, pKTH50) may also be used as vectors for producing the polypeptides of the present invention.

In order to express proteins in animal cells, such as CHO, COS, and NIH3T3 cells, the vector preferably has a promoter necessary for expression in such cells, for example, an SV40 promoter (Mulligan et al. (1979) Nature 277:108), MMLV-LTR promoter, EF1αpromoter (Mizushima et al. (1990) Nucleic Acids Res. 18:5322), CMV promoter, etc. It is even more preferable that the vector also carries a marker gene for selecting transformants (for example, a drug-resistance gene enabling selection by a drug such as neomycin and G418). Examples of vectors with such characteristics include pMAM, pDR2, pBK-RSV, pBK-CMV, pOPRSV, pOP13, and such.

In addition, to stably express a gene and amplify the gene copy number in cells, CHO cells having a defective nucleic acid synthesis pathway can be introduced with a vector containing a DHFR gene (for example, pCHOI) to compensate for the defect, and the copy number may be amplified using methotrexate (MTX). Alternatively, a COS cell, which carries an SV40 T antigen-expressing gene on its chromosome, can be transformed with a vector containing the SV40 replication origin (for example, pcD) for transient gene expression. The replication origin may be derived from polyoma viruses, adenoviruses, bovine papilloma viruses (BPV), and such. Furthermore, to increase the gene copy number in host cells, the expression vector may contain, as a selection marker, an aminoglycoside transferase (APH) gene, thymidine kinase (TK) gene, *E. coli* xanthine guanine phosphoribosyl transferase (Ecogpt) gene, dihydrofolate reductase (dhfr) gene, and such.

In the present invention, combined use of an anti-HLA class I antibody with an interferon means administering or using an anti-HLA class I antibody together with an interferon (hereinafter, simply referred to as "administering"), and there is no limitation regarding the order of administration and interval between administrations. The order of administration of an interferon and an anti-HLA class I antibody in the present invention may be any one order of, administering an interferon and then administering an anti-HLA class I antibody, administering an interferon and an anti-HLA class I antibody simultaneously, or administering an anti-HLA class I antibody and then administering an interferon. However, a preferable order includes administering an interferon and then administering an anti-HLA class I antibody, or administering an interferon and an anti-HLA class I antibody simultaneously, and a more preferable order is administering an interferon and then administering an anti-HLA class I antibody.

When administering an interferon and then administering an anti-HLA class I antibody, the interval between the two administrations is not particularly limited, and can be set upon consideration of factors such as route ofadministration and dosage form. An example of an administration interval would ordinarily be 0 to 72 hours, preferably 0 to 24 hours, and more preferably 0 to 12 hours.

Herein, an effect (action) of an anti-HLA class I antibody refers to a biological action that arises as a result of antigen-antibody binding. Specific examples of the biological action include cell death induction, apoptosis induction, cell growth suppression, cell differentiation suppression, cell division suppression, cell growth induction, cell differentiation induction, cell division induction, and cell cycle regulation and the like. In the present invention, the above-mentioned activity that is to be enhanced by an interferon may be any activity so long as it is the activity possessed by an anti-HLA class I antibody; however, cell death induction effect and cell growth suppression effect are preferred.

Cells that become a target of the above-mentioned actions such as cell death induction and cell growth suppression, are not particularly limited, though hematopoietic cells and non-adherent cells are preferred. Specific examples of hematopoietic cells include lymphocytes (B cells, T cells), neutrophils, eosinophils, basophils, monocytes (preferably activated peripheral blood mononuclear cells (PBMC)), and multiple myeloma (myeloma or MM) cells, and are preferably lymphocytes (B cells, T cells) and myeloma cells, and most preferably T cells and B cells (particularly activated B cells or activated T cells) and myeloma cells. "Non-adherent cells" refers to cells that, when cultured, grow in a non-adherent state without adhering to the surface of culturing vessels such as glass or plastic. On the other hand, "adherent cells" refers to cells that, when cultured, adhere to the surface of culturing vessels such as glass or plastic.

In the present invention, simultaneous administration of an anti-HLA class I antibody and an interferon can treat or prevent diseases such as tumors including hematopoietic tumors (specific examples include leukemia; myelodysplastic syndrome; malignant lymphoma; chronic myeloid leukemia; plasmacytic disorders such as myeloma, multiple myeloma, and macroglobulinemia; and myeloproliferative diseases such as polycythemia vera, essential thrombocythemia, and idiopathic myelofibrosis; and such), and autoimmune diseases (specific examples include rheumatism, autoimmune hepatitis, autoimmune thyroiditis, autoimmune bullosis, autoimmune adrenocortical disease, autoimmune hemolytic anemia, autoimmune thrombycytopenic purpura, autoimmune atrophic gastritis, autoimmune neutropenia, autoimmune orchitis, autoimmune encephalomyelitis, autoimmune receptor disease, autoimmune infertility, Crohn's disease, systemic lupus erythematosus, multiple sclerosis, Basedow's disease, juvenile diabetes, Addison's disease, myasthenia gravis, lens-induced uveitis, psoriasis, and Behchet's disease). A preferred disease to be targeted is hematopoietic tumors, and particularly preferred disease to be targeted is multiple myeloma.

An anti-HLA class I antibody can be made into a single pharmaceutical composition together with an interferon. Furthermore, an anti-HLA class I antibody can be made into a pharmaceutical composition that is characterized by being used in combination with an interferon. More specifically, an anti-HLA class I antibody can be used to prepare a "pharmaceutical composition comprising an anti-HLA class I antibody and a pharmaceutically acceptable carrier, wherein the composition is used in combination with an interferon". An interferon can be made into a pharmaceutical composition that is characterized by being used in combination with an anti-HLA class I antibody. More specifically, an interferon can be used to prepare a "pharmaceutical composition comprising an interferon and a pharmaceutically acceptable carrier, wherein the composition is used in combination with an anti-HLA class I antibody".

The above-mentioned pharmaceutically acceptable carrier refers to a material that does not have the above mentioned activity in itself, and that is pharmaceutically acceptable and can be administered together with the above-mentioned pharmaceutical agent. Pharmaceutically acceptable carriers are used for example as stabilizers, buffers, stabilizing agents, preservatives, excipients, suspending agents, emulsifiers, and solubilizers.

For example, about 0.2% gelatin or dextran, 0.1 to 1.0% sodium glutamate, approximately 5% lactose, or approximately 2% sorbitol, or such may be used as a stabilizer, without being limited thereto. As preservatives, approximately 0.01 % thimerosal, approximately 0.1 % beta-propiolactone and such may be used, without being limited thereto.

When preparing injections, pH-adjusting agents, buffers, stabilizers, preservatives or such are added as necessary to prepare subcutaneous, intramuscular, and intravenous injections by common procedures. An injection can be prepared as a solid preparation for formulation immediately before use by freeze-drying a solution stored in a container. A single dose can be stored in a container or multiple doses may be stored in a same container.

In the present invention, the administration may be oral or parenteral administration. Oral administration includes administration in the form of oral agents, and the dosage form for oral agents can be selected
from granules, powders, tablets, capsules, dissolved agents, emulsion, suspension, and such. Parenteral administration includes administration in an injectable form; examples of an injection include subcutaneous injection, intramuscular injection, and intraperitoneal injection. Methods for administering an injection may be those for local administration targeting a part within the body (a tissue in an organ or such) of a subject organism, or may be those for intravascular administration to circulate the present invention's antibodies throughout the body of an organism. Administrations targeting multiple sites may be concomitantly carried out. Furthermore, effects of the methods of the present invention can be accomplished by introducing a gene encoding an antibody to be administered into a living organism using gene therapy techniques. Methods for introducing into and expressing in a living body a gene encoding a protein that produces the effect of the present invention's method are well known. The antibodies of the present invention can be administered locally to a region to be treated. For example, the antibodies can be administered by local infusion or by the use of a catheter during surgery, or by targeted gene delivery of a sequence encoding a therapeutic agent.

Dosage varies depending on the age, sex, body weight, and symptoms of patients, and on therapeutic effect, method of administration, processing time, types of active ingredients included in the pharmaceutical compositions, and such; nevertheless, an ordinary dosage for an adult is in the range of 0.1 mg to 1000 mg per administration. However, since dosages may vary depending on various conditions, one that is smaller than the above-mentioned dosage may be sufficient in some cases, while one exceeding the above-mentioned range may be required in other cases.

All references cited herein are incorporated by reference into this description.

### Examples

Herein below, the present invention will be specifically described with reference to Examples, but it is not to be construed as being limited thereto.

### [Example 1] Effects of interferons (IFNs) on 2D7-DB-mediated cell injury

Since cell surface expression of HLA class I is enhanced by IFN stimulation, effects of IFNs on HLA-A expression and 2D7-DB-mediated cytotoxicity were examined using IM-9 cells. After treating IM-9 cells with IFN-α (100 U/mL, OIF 2,500,000 IU, Otsuka Pharmaceutical) or IFN-γ (100 U/mL, Ogamma 100, Otsuka Pharmaceutical) for 48 hours, flow cytometry was performed. The amino acid sequence and nucleotide sequence of IFN-α mentioned above are shown in SEQ ID NOs: 1 and 2, respectively; and the amino acid sequence and nucleotide sequence of IFN-γ mentioned above are shown in SEQ ID NOs: 3 and 4, respectively. Flow cytometry analyses demonstrated that HLA-A expression was up-regulated after IFNα or IFNγ stimulation (Fig. 1A).

Furthermore, IM-9 cells were treated with IFN-α (100 U/mL) or IFN-γ (100 U/mL) for 48 hours, then cultured with 2D7-DB (1 µg/mL) for 24 hours, and cell viability was determined by WST-8 assays. As a result, cell death activity was found to be increased by the combination of 2D7-DB and IFN-α or IFN-γ (Fig. 1B).

### [Reference Example 1] Expression of HLA class I in tumor cell lines

Reactivity of 2D7 mAb in tumor cell lines and myeloma cells derived from multiple myeloma patients were assessed by flow cytometry.

The tumor cell lines used were prepared as follows. The myeloma cell line RPMI 8226, B lymphoblastic cell lines (IM-9, Raji, HS-Sultan, and Daudi), the T lymphoblastic cell line CEM, the chronic myeloid leukemia cell line K562, the lung adenocarcinoma cell line A549, the colon adenocarcinoma cell line COLO 201, and the gastric adenocarcinoma cell line MKN-1 were obtained from the Health Science Resources Bank (Osaka, Japan). The myeloma cell line U266-B1, the B lymphoblastic cell line ARH-77, the T-lymphoblastic cell line Jurkat, the breast cancer cell line MCF-7, the hepatocellular carcinoma HepG2, the clear cell renal cell carcinoma Caki-2, and the malignant melanoma MeWo were obtained from the American Type Culture Collection (Manassas, Virginia). The EBV-transformed B-cell line YN was a gift from Dr. Yasuhiko Nishioka (The University of Tokushima). These cell lines were cultured in an RPMI-1640 medium (Sigma-Aldrich, St. Louis, Missouri) supplemented with 10% fetal calf serum (Gibco BRL, Grand Island, New York) and penicillin (100 U/mL) under a humidified atmosphere of 5% CO₂ at 37°C.

Patients' MM cells were prepared as follows. First, clinical disease stage classification and diagnosis of MM patients were performed according to the Durie and Salmon classification (Durie, B. G, and S. E. Salmon. 1975. "A clinical staging system for multiple myeloma. Correlation of measured myeloma cell mass with presenting clinical features, response to treatment, and survival." Cancer, 36:842-854). Bone marrow samples were collected from patients after obtaining informed consent upon approval by the ethics committee of The University of Tokushima,. The bone marrow mononuclear cells (BMMCs) were separated by Ficoll-Hypaque (density of 1.077) centrifugation. Patient-derived MM cells were further purified from BMMCs using CD138 microbeads (Miltenyi Biotec, Auburn, California).

Cell surface expression analysis of HLA class I by flow cytometry was performed as follows using FITC-labeled 2D7 mAb or FITC-labeled anti-HLA-ABC common region mAb (Chemicon, San Diego, California). The cells (5 x 10⁵ cells) were washed twice with PBS, and then incubated on ice for 40 minutes with FITC-labeled 2D7 mAb or FITC-labeled anti-HLA-ABC common region mAb. MM patient-derived BMMCs were incubated on ice for 15 minutes with 2% γ globulin diluted in PBS, and stained with FITC-labeled 2D7 mAb and PE-labeled anti-CD38 mAb (BD Bioscience, San Jose, California) for myeloma cell gating. Next, the cells were washed twice with PBS, and analyzed using an EPICS XL flow cytometer (Beckman Coulter, Japan). In several experiments, the cells were stimulated for 48 hours with IFNα or IFNγ prior to HLA class I expression analysis. HLA class I expression levels were evaluated as GeoMFI using the CellQuest software (BD Biosciences).

**Table 1**

| Cell line | Lineage | Expression (geometric mean fluorescence intensity) | | Cytotoxicity by 2D7-DB (%) |
|---|---|---|---|---|
| | | 2D7 | HLA-ABC | |
| ARH77 | B-lymphoblastic cells | 3727 | 6148 | 58 |
| YN | EBV-transformed B-cells | 804 | 2399 | 66 |
| IM-9 | B-lymphoblastic cells | 662 | 1728 | 75 |
| RPMI 8226 | myeloma cells | 509 | 2194 | 20 |
| U266 | myeloma cells | 340 | 782 | 29 |
| Raji | B-lymphoblastic cells | 297 | 1544 | 5 |
| HS-Sultan | B-lymphoblastic cells | 285 | 554 | 6 |
| Jurkat | T-lymphoblastic cells | 111 | 585 | 30 |
| COLO 201 | colon adenocarcinoma cells | 105 | 106 | 4 |
| CEM | T-lymphoblastic cells | 72 | 166 | 2 |
| MKN-1 | gastric adenocarcinoma cells | 49 | 136 | 0 |
| A549 | lung adenocarcinoma cells | 37 | 302 | 10 |
| HepG2 | hepatocellular carcinoma | 33 | 196 | 0 |
| MCF-7 | breast cancer cells | 29 | 5 | 5 |
| MeWo | malignant melanoma | 21 | 86 | 8 |
| Caki-2 | clear cell renal cell carcinoma | 13 | 32 | 0 |
| K562* | chronic myeloid leukemia cells | 11 | 51 | 2 |
| Daudi† | B-lymphoblastic cells | 2 | 5 | 2 |

As shown in Table 1, in MM cell lines and B-lymphoblastic cell lines such as ARH-77, YN, IM-9, RPMI 8226, and U226, HLA class I recognized by 2D7 mAb or anti-HLA-ABC common region mAb was expressed at high levels. The levels of HLA class I expression were relatively low in T cell lines and cancer cell lines of other lineages. Since K562 cells lack the HLA-A and HLA-B genes (Johnson, D. R. 2000. "Differential expression of human major histocompatibility class I loci: HLA-A, -B, and -C" Hum Immunol., 61:389-396) they did not show 2D7 antigen (HLA-A) expression but showed a low level of HLA class I expression. Daudi cells do not produce β2-microglobulin (Rosa, F., M. Fellous, M. Dron, M. Tovey, and M. Revel. 1983. "Presence of an abnormal beta 2-microglobulin mRNA in Daudi cells: induction by interferon" Immunogenetics, 17:125-131) and did not express HLA class I molecules on their cell surface.

Next, HLA-A expression in BMMCs of MM patients was examined. BMMCs were stained with FITC-labeled 2D7 mAb and phycoerythrin (PE)-labeled anti-CD38 mAb. After gating the cells according to their side-scattered light profile and CD38 expression, each of the cell populations comprising myeloma cells, myeloid cells, and lymphocytes was analyzed for HLA-A expression. As shown in Fig. 2A, HLA-A expression level was higher in myeloma cells as compared to normal myeloid cells and lymphocytes. When the BM samples of 22 MM patients were analyzed, the HLA expression level in myeloma cells expressed as geometric mean fluorescence intensity (GeoMFI, 1309 ± 1130 [mean ± SD]) was significantly higher as compared to each of myeloid cells (201 ± 209) and lymphocytes (91 ± 44) (Fig. 2B). HLA-A expression in MM cells was not related to the clinical disease stages of MM. Furthermore, from two-color flow cytometry, CD34⁺ cells in a peripheral blood stem cell collection were shown to express HLA-A at a low level (GeoMFI, 109 ± 16, n = 3).

### [Reference Example 2] Effects of HLA class I antibodies on myeloma cells

Cytotoxic effects of anti-HLA class I mAb on MM cell lines and patient-derived MM cells were examined.

Cytotoxicity assays were performed as follows. First, a patient's MM cells (2 x 10⁶ cells/mL) or cell lines (5 x 10⁵ cells/mL) were incubated in an RPMI 1640 medium supplemented with 10% FCS at 37°C for 30 minutes with various concentrations of anti-HLA class I mAbs, such as 2D7, W6/32, B9.12.1, and JOAN-1, and then incubated for 24 hours with 10 µg/mL goat anti-mouse IgG antibody F(ab')₂ (Jackson ImmunoResearch Laboratories, West Grove, Pennsylvania). Cell viabilities were measured by WST-8 assays (Kishida Chemical, Osaka, Japan) for the cell lines, and by Trypan Blue dye exclusion assays for patient-derived primary MM cells. Apoptosis induction was assessed by flow cytometric analysis with Annexin V and PI staining (MEBCYTO apoptosis kit, MBL, Nagoya, Japan).

Anti-HLA class I mAbs, such as W6/32, B9.12.1, and JOAN-1, did not affect cell viability when used alone (data not shown). However, in the presence of a secondary goat anti-mouse IgG antibody, these anti-HLA class I mAbs induced aggregation of MM cell lines, B-lymphoblastic cell lines (ARH-77, IM-9, and U266), and the patient's MM cells in a dose-dependent manner. Aggregation of cells caused cell death, which was confirmed by a WST-8 assay or Trypan Blue exclusion assay. Cell viabilities 24 hours after cross-linking each of these anti-HLA class I mAbs with the secondary antibody are shown in Fig. 3A for IM-9 cells and the patient's MM cells. 2D7 mAb inhibited the cell viability of IM-9 more efficiently than W6/32, B9.12.1, and JOAN-1. Cell viability of the newly isolated MM cells was also inhibited by 2D7 mAb in the presence of a secondary antibody. This cell aggregation and cell death took place rapidly within one hour and reached a maximum six hours later, as reported previously (Non-Patent Document 7).

### [Reference Example 3] Cytotoxic effects of 2D7-DB

Next, cytotoxic effects of single chain Fv-type 2D7 mAb (2D7-DB) on MM cell lines and patient-derived MM cells were assessed. Cell injury assays were basically performed as described above, but without addition of a secondary goat anti-mouse IgG antibody.

In both IM-9 cells and a patient's MM cells, cell viability decreased more remarkably by 2D7-DB than by 2D7 mAb (Fig. 3A). Importantly, the newly isolated MM cells were more sensitive to 2D7-DB than the MM cell lines. Morphological studies revealed that most of the patient's MM cells showed cytoplasmic vacuolar degeneration within 24 hours of 2D7-DB treatment (Fig. 3B). The effects of 2D7-DB on MM cells purified from six patients were analyzed, and it was found that the cell viability after 24 hours of treatment decreased to 51 ± 18.4% (mean ± SD) in the case of 0.1 µg/mL 2D7-DB, and to 26 ± 17.6% (mean ± SD) in the case of 1 µg/mL 2D7-DB (Fig. 3C). Sensitivity to 2D7-DB was associated with the HLA-A expression level in the patient's MM cells. In contrast, cell viabilities of normal myeloid cells or lymphocytes were not affected by such amounts of 2D7-DB, as reported previously.

To determine the effects of 2D7-DB on other hematopoietic cells, expression of HLA-A in hematopoietic cells in the bone marrow of an MM patient and effects of 2D7-DB on these hematopoietic cells were examined. Total BMMCs were treated with 2D7-DB for 24 hours, and then the cell populations were examined by flow cytometry. As shown in Fig. 4A, CD38⁺ MM cells were selectively and drastically decreased in all of the BMMC fractions from MM patients. The remaining cells were analyzed with anti-HLA-ABC mAb, and it was found that only the myeloma cell population overexpressing HLA class I was selectively eliminated (Fig. 4A). These findings were also confirmed from morphological studies showing that 2D7-DB selectively induces death of MM cells without affecting normal myeloid cells or lymphocytes (Fig. 4B).

Furthermore, effects of 2D7-DB on hematopoietic progenitor cells were determined by colony assays. A MethoCult assay (Stem Cell Technologies, Vancouver, Canada) was used to measure granulocyte-macrophage colony forming units and erythroid burst-forming units. According to the assay on hematopoietic progenitor cells, 2D7-DB (1 µg/mL) did not significantly inhibit increase of granulocyte-macrophage colony forming units and erythroid burst-forming units (data not shown).

Since interactions between MM cells and bone marrow microenvironment play important roles in the growth and survival of MM cells, effects of 2D7-DB on MM cells and bone marrow stromal cells were examined. Newly isolated MM cells were cultured in a 24-well plate in the presence of bone marrow stromal cells, and then treated for 24 hours with 2D7-DB. As shown in Fig. 4C, treatment with 2D7-DB caused efficient removal of a patient's MM cells adhered to bone marrow stromal cells; however, normal stromal cells were not significantly affected. Similarly, cell injury by 2D7-DB was not observed in adherent human umbilical vein endothelial cells (HUVEC) (data not shown).

### [Reference Example 4] In vivo antitumor effects of 2D7-DB

Finally, *in vivo* efficacy of 2D7-DB was evaluated in human myeloma xenograft models. First, to evaluate the *in vivo* cytotoxic activity of 2D7-DB, human myeloma cells were xenografted into severe combined immunodeficient (SCID) mice, in a similar manner as described in literature (S. Ozaki, M. Kosaka, S. Wakatsuki, M. Abe, Y. Koishibara, and T. Matsumoto. 1997. "Immunotherapy of multiple myeloma with a monoclonal antibody directed against a plasma cell-specific antigen, HM1.24" Blood, 90:3179-3186). To eradicate the remaining natural killer cells, 10 µL of rabbit anti-asialo GM1 antiserum (Wako Chemicals, Osaka, Japan) was intraperitoneally injected to the above-mentioned SCID mice one day before tumor inoculation. Next, ARH-77 cells (6 x 10⁶ cells) were inoculated to the above-mentioned SCID mice by intravenous injection via the tail vain, and the mice were divided into two groups, each consisting of seven mice. One, two, and three days after the ARH-77 cell inoculation, the SCID mice were subjected to intravenous injection of 8 mg/kg of 2D7-DB or phosphate buffered saline (PBS) twice a day, and the survival time was monitored. On the 24th day, human M protein level in the serum was measured by enzyme-linked immunosorbent assay (ELISA).

As shown in Fig. 5A, 2D7-DB-treated mice showed a marked decrease in the serum M protein level on the 24th day (20 ± 19 µg/mL, mean ± SD) as compared to that of the control mice (74 ± 66 µg/mL). Furthermore, treatment with 2D7-DB markedly lengthened the survival time of mice carrying disseminated ARH tumors (Fig. 5B). Toxicity associated with 2D7-DB treatment, which is indicated from the body weight of these mice, was not observed. These results suggest that 2D7-DB provides antitumor activity *in vivo.*

### [Reference Example 5] Antitumor mechanism of 2D7-DB

It is strongly suggested that in the antitumor mechanism of minibody-type HLA antibody (2D7-DB), the antitumor effect may be caused through the disruption of the intracellular actin cytoskeletal system by 2D7-DB bound to HLA class IA (Patent Document 1). This time, the present inventors further examined the antitumor mechanism of 2D7-DB. First, 60 minutes of pretreatment was performed using various signal transduction inhibitors, and their effects on cell injury of the myeloma cell line RPMI 8226 after 2D7-DB (1 µg/mL) addition were examined (Fig. 6A). Caspase inhibitor (z-VAD-fmk, 5 µM), MAP kinase inhibitor (PD 98059, 10 µM), and PI-3 kinase inhibitor (Wortmannin, 10 µM) did not show effects on cytotoxicity of 2D7-DB. However, Clostridium difficile toxin B (TcdB, 10 pM) which is a Rho GTPase inhibitor, and cytochalasin D (1 µM) and latrunculin A (0.1 µM) which are actin polymerization inhibitors almost completely suppressed the cytotoxicity of 2D7-DB. These results suggest that cell injury by 2D7-DB involves actin polymerization mediated by Rho GTPase activity.

Furthermore, actin staining of the cells was performed to examine the localization of 2D7-DB (green) and actin (red) (Fig. 6B). Ten minutes after treatment of the myeloma cell line ARH-77 cells with 2D7-DB (1 µg/mL), a marked aggregation of actin in the cytoplasm was observed, and in some cells, actin flowed out due to cell injury. However, when 30 minutes of pretreatment was performed with an actin polymerization inhibitor, latrunculin A (0.1 µM), actin stayed localized at the periphery of the cells even after 2D7-DB addition and aggregation was not observed. On the other hand, actin aggregation due to 2D7-DB was not observed in the bone marrow stromal cell line KM102 expressing HLA-A. Thus, actin aggregation effect and cytotoxic activity by 2D7-DB were considered specific to myeloma cells and lymphoid cells, and while 2D7-DB is useful for myeloma and autoimmune diseases, it was considered to have little effect on normal tissues.

Next, signal transduction associated with cell injury by 2D7-DB was examined by Western blotting (Fig. 7ABC). In KM102 cells, FAK, Akt, or ERK was detected to be phosphorylated after stimulation with 2D7-DB (1 µg/mL), and their signal transduction pathways were considered to have been activated. On the other hand, in RPMI 8226 cells, activation of these signal transduction pathways were not observed, and this showed that stimulation with 2D7-DB has different effects depending on the cell type. Furthermore, since FAK, Akt, and ERK are generally signaling pathways involved in cell survival, 2D7-DB may be acting to promote cell growth in KM102 cells.

Finally, the presence or absence of apoptosis induction by 2D7-DB was examined (Fig. 7D). When RPMI 8226 was stimulated with 2D7-DB, characteristics of apoptosis such as caspase activation and PARP cleavage were not observed. Accordingly, cell injury by 2D7-DB was different from the so-called apoptosis and was considered to be due to the mechanism called actin aggregation.

Interferon is considered to exhibit cytotoxic action by inducing apoptosis, and signaling molecules such as Jak-Stat, MAP kinase, and PI3 kinase have been reported to be involved in this process (Documents: J. Interferon Cytokine Res. 2005 Dec; 25(12):799-810 and Apoptosis. 2003 Jun; 8(3):237-49). On the other hand, since 2D7-DB destroys target cells by actin aggregation, molecules involved in such signal transduction were found not to be activated, as shown in Fig. 7. As described, the mechanisms of cell injury induced by 2D7-DB and interferon are different, and combination therapy using the two seemed to show potentiating effects.

### Industrial Applicability

The present invention provided novel pharmaceutical compositions comprising an anti-HLA class I antibody. The pharmaceutical compositions of the present invention strongly enhance the effects of anti-HLA class I antibodies, such as cell death induction, through combined use of anti-HLA class I antibodies and interferons. The pharmaceutical compositions of the present invention can exert an increasingly high drug efficacy due to anti-HLA class I antibody action.

## Claims

1. A pharmaceutical composition comprising an interferon and an anti-HLA class I antibody as active ingredients.

2. A pharmaceutical composition comprising an anti-HLA class I antibody as an active ingredient, wherein the composition is used in combination with an interferon.

3. A pharmaceutical composition comprising an anti-HLA class I antibody as an active ingredient, wherein the composition is administered simultaneously with an interferon.

4. A pharmaceutical composition comprising an anti-HLA class I antibody as an active ingredient, wherein the composition is administered after administration of an interferon.

5. The pharmaceutical composition of any one of claims 1 to 4, wherein the anti-HLA class I antibody is an anti-HLA-A antibody.

6. The pharmaceutical composition of any one of claims 1 to 5, wherein the anti-HLA class I antibody is a minibody.

7. A cell death-inducing agent comprising an interferon and an anti-HLA class I antibody as active ingredients.

8. The cell death-inducing agent of claim 7, wherein the anti-HLA class I antibody is an anti-HLA-A antibody.

9. The cell death-inducing agent of claim 7 or 8, wherein the anti-HLA class I antibody is a minibody.

10. A pharmaceutical composition comprising an interferon, wherein the composition is used in combination with an anti-HLA class I antibody.

11. A pharmaceutical composition comprising an interferon, wherein the composition is administered simultaneously with an anti-HLA class I antibody.

12. A pharmaceutical composition comprising an interferon, wherein the composition is administered before administration of an anti-HLA class I antibody.

13. An anti-HLA class I antibody potentiator comprising an interferon as an active ingredient.
